# EUROPEAN PATENT APPLICATION

(11) **EP 4 112 086 A1**
(43) Date of publication of application: **04.01.2023**
(21) Application number: 21760472.7
(22) Date of filing: 24.02.2021
(51) Int. Cl.: A61L 2/22, B05B 9/04, A61L 9/14

(54) **MOBILE HYGIENE UNIT**

(30) Priority: 26.02.2020 RU 2020108362
(71) Applicant: Nexigreen Solutions Ltd, Limassol (CY)
(72) Inventor: VELKOVICH, Mihail Abramovich, Sankt-Peterburg, 191186 (RU); SHMATKOV, Aleksandr Alekseevich, Sankt- Peterburg, 197373 (RU); RUCHYEV, Andrei Andreevich, Sankt-Peterburg, 197373 (RU)
(74) Representative: Betten & Resch
(86) International application number: PCT/RU2021/000079
(87) International publication number: WO 2021/173038

(57) **Abstract**

The invention relates to automated devices for producing aerosols of liquid disinfectants and for spraying same. A mobile hygiene unit comprises a housing (1) containing a disinfectant spraying unit (2) with a spraying nozzle (3), a tank (4) containing disinfectant, a compressor (5), and a control unit (6). It further comprises a mechanism for regulating the droplet size distribution of the aerosol, including a pump (7), an electromagnetic valve (8), and a dosing nozzle (9) with an internal cylinder. The disinfectant spraying unit (2) comprises a shaped nozzle (3) having a nozzle head (10) of axisymmetric cross-section attached thereto, and a mixing chamber (11) for mixing disinfectant with compressed air, having two threaded inlets for a pneumatic line (12) and a hydraulic line (13), said inlets being arranged at an angle of 180°. The dosing nozzle (9) contains an internal cylinder and a rod with a threaded tip. The mobile hygiene unit generates an aerosol in four droplet size distribution modes: dry fog (from 3.5 to 10 pm), wet fog (from 10 to 30 pm), mist (from 30 to 50 pm), and spray (from 50 to 100 pm). The invention allows highly precise regulation of the droplet size distribution of the aerosol in each mode without the need to change the spraying nozzle.

## Description

### PERTINENT ART

The claimed invention relates to automated devices for producing aerosols of liquid disinfectants and for spraying same and can be used for disinfection of enclosed spaces of various purposes, including in medical organizations, agriculture, pharmaceutical enterprises and high-precision industries.

### PRIOR ART

Currently, devices with an aerosol method of applying disinfectants are used for effective disinfection of enclosed spaces for various purposes.

There is a device for aerosol spraying of liquid, comprising a tank with liquid installed in the housing and at least one pneumatic nozzle, the air piping thereof being connected to a compressed air supply system including an air filter and compressor, whereas the liquid piping is connected to the tank with liquid. Herewith, the device in question is additionally equipped with an air duct installed in the housing with a fan whose axis is not parallel to the axis of the nozzle tip (RF patent No. 185062, 2018).

The disadvantage of this device is the lack of regulation of droplet size distribution of the aerosol and the lack of automation of the device.

There is also a device for aerosol disinfection, comprising a housing in which a spraying unit of a disinfectant is installed, including a spraying nozzle communicating via a fluid with a tank with a disinfectant, and a compressor connected to a spraying nozzle, as well as a control unit. The device in question also contains a controller made so as to allow selective activation of the drive unit to lock and unlock the access door to the disinfected area, whereas the control unit interacts with the controller and initiates an automatic disinfecting cycle, during which the disinfectant is sprayed through the spraying nozzle when the access door to the treated area is blocked. The device in question is compact and easy to move from one room to another (US patent application US2019167829 (A1), 2019). This device is taken as the closest analog.

The disadvantage of this device is the lack of regulation of droplet size distribution of the aerosol and the inability to implement all the modes of the aerosol method, as well as the need to change the spray nozzles depending on the disinfectants used.

### DISCLOSURE OF THE INVENTION

The technical issue solved by the claimed invention consists in the creation of a mobile hygienic unit for aerosol disinfection of enclosed spaces, providing high-precision regulation of droplet size distribution of the aerosol in all modes of the aerosol method using any disinfectants, including chlorine dioxide, without changing the spray nozzle, as well as in expanding the range of technical means of automated aerosol disinfection.

The technical result provided by the claimed invention consists in the implementation of its purpose by the invention.

The specified technical result is achieved due to the fact that the mobile hygiene unit, comprising a housing containing a spraying unit of the disinfectant with a spraying nozzle and a chamber for mixing the disinfectant with compressed air, a tank with a disinfectant, a compressor and a control unit. It additionally contains a mechanism for regulating droplet size distribution of the aerosol, including a dozing nozzle with an internal cylinder and a rod with a threaded tip at the same time, the mixing chamber of the disinfectant with compressed air interacting with the dosing nozzle, and the inner cylinder of the dozing nozzle being designed so to make it possible for the disinfectant and compressed air to move in the mixing chamber, in addition, the spraying nozzle is equipped with a nozzle head of axisymmetric cross-section.

In addition, the mobile hygiene unit can be equipped with antistatic swivel wheels.

The mobile hygiene unit can also include a light alarm unit and a sound alarm unit as well.

The mobile hygiene unit can be equipped with a remote control as well.

The mobile hygiene unit can also comprise the spraying unit of the disinfectant and the dosing nozzle made of an anticorrosive chemically resistant material.

### A BRIEF DESCRIPTION OF THE FIGURES OF THE DRAWINGS

The invention is illustrated by drawings, which depict:
- Figure 1 shows a general view of the mobile hygiene unit;
- Figure 2 shows a diagram of the spraying unit and the mechanism for regulating the droplet size distribution of the aerosol.

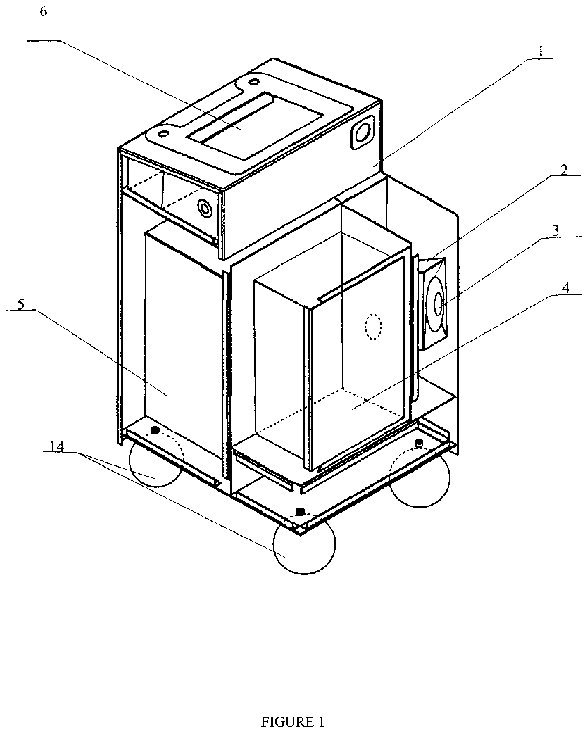

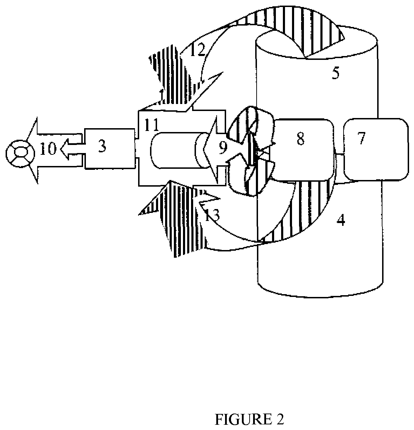

According to Figures 1-2, the mobile hygiene unit comprises housing 1, which contains spraying unit 2 of a disinfectant with spraying nozzle 3, tank 4 with a disinfectant, compressor 5 and control unit 6. In addition, the mobile hygiene unit contains a mechanism for regulating the droplet size distribution of the aerosol, including pump 7, electromagnetic valve 8 and dosing nozzle 9 with an internal cylinder.

Housing 1 is made of an anticorrosive chemically resistant material, for example, aluminum alloy or plastic, and contains vibration mounts in the lower part from the inside, protecting the mobile hygiene unit from vibrations during operation. In addition, housing 1 is equipped with a connector for a power cable, with which the unit is connected to the power supply network.

Spraying unit 2 of the disinfectant is detachable in housing 1, for example, with bolts, and includes shaped nozzle 3, for example, Laval nozzle, with nozzle of axisymmetric cross-section 10 attached to it and a chamber for mixing the disinfectant with compressed air, which is a hollow tank with two threaded inlets for pneumatic line 12 and hydraulic line 13, said inlets being arranged at an angle of 180 °.

Pneumatic line 12 consists of pipes, threaded connections, barb fittings and a reinforced double-layer pipe and is designed to supply compressed air from compressor 5 to spraying unit 2.

Hydraulic line 13 consists of pipes and threaded connections and is designed to supply disinfectant from container 4 to spraying unit 2.

Pneumatic line 12 and hydraulic line 13 are made of a synthetic polymer, for example, polytetrafluoroethylene (PTFE).

Tank 4 with the disinfectant is equipped with a screw cap with a headset for liquid intake and is installed in the lower part of housing 1.

Compressor 5 is of oil-free air type and provides pressure from 3 to 10 bar. Compressor 5 is equipped with a cooling system and is installed on the lower panel in housing 1, fixed with vibration mounts.

Pump 7 relates to chemically resistant membrane pumps and is designed to regulate the amount of disinfectant supplied. Electromagnetic valve 8 relates to microvalves for aggressive agents and is designed to hold fix the amount of disinfectant measured by pump 7. Dosing nozzle 9 contains an inner cylinder and a rod with a threaded tip. Pump 7, electromagnetic valve 8 and dosing nozzle 9 are detachable in housing 1, for example, with bolts.

All elements of spraying unit 2 and dosing nozzle 9 are made of an anticorrosive chemically resistant material, for example, stainless steel on a chromium-nickel-molybdenum base, which is resistant to hydrogen peroxide up to 34%, chlorine dioxide up to 3.9%, peracetic acid up to 9.9%, quaternary ammonium compounds, guanidine derivatives up to 49%, alkylamines up to 70%, chloractive compounds up to 20%, aldehydes up to 50%, alcohols up to 95%, phenol and its derivatives up to 20%.

Control unit 6 contains a display, relay modules and a computer to ensure the functioning of the mobile hygiene unit. With the help of the program installed on the computer, aerosol disinfection is carried out automatically.

The mobile hygiene unit smoothly generates aerosol with a droplet size distribution in the range from 3.5 to 100 micrometers in four modes: dry fog (from 3.5 to 10 micrometers), wet fog (from 10 to 30 micrometers), mist (from 30 to 50 micrometers) and spray (from 50 to 100 micrometers).

The mobile hygiene unit can be equipped with antistatic swivel wheels 14, which are fixed in two positions - in the direction of movement and stopping on the spot. This allows to operate the mobile hygiene unit when moving and securely fix it in place.

The mobile hygiene unit can also include a light alarm unit for light warning accompaniment of the aerosol disinfection process and a sound alarm unit for warning about the beginning of this process.

In addition, the mobile hygiene unit can be equipped with a remote control with Wi-Fi and Bluetooth support for monitoring processes and remote control of the unit.

### EMBODIMENT OF INVENTION

The mobile hygiene unit operates as follows.

The user turns on the mobile hygiene unit, launches the unit operation program, selects the disinfecting mode and disinfectant depending on the purpose of the room and the type of pathogenic microorganisms and starts the disinfecting process. If there are light and sound alarm units, a warning is received about the need to leave the disinfected room. If there is a remote control, the user leaves the disinfected room with this remote control. After 30 seconds from the start of the disinfecting process, compressor 5 supplies compressed air with a predetermined pressure and speed in accordance with the spraying mode to chamber 11 through the threaded inlet for pneumatic line 12. Simultaneously, according to a signal from control unit 6, the amount of disinfectant measured by pump 7 and fixed by electromagnetic valve 8 enters chamber 11 through the threaded inlet for hydraulic line 13 at a predetermined speed in accordance with the spraying mode. Thus, the disinfectant and compressed air flow into the passage from the opposite side to each other and at an angle to the inner cylinder of dosing nozzle 9, accelerating, forming and unwinding the aerosol mixture. The amount of the mixture is regulated by dosing nozzle 9 with an internal cylinder, which, when the rod with the tip is rotated, moves in and out of chamber 11, reducing or increasing the volume of the outgoing aerosol per unit of time. The aerosol mixture from chamber 11 enters spraying nozzle 3, where the swirling flow is leveled, accelerated again and enters nozzle 10. Passing through the axisymmetric section of nozzle 10, the flow accelerates with the formation of a torch in the form of a hollow cone, which enters the disinfected room. Such multi-stage aerosol separation, precisely measured at each stage, makes it possible to achieve smooth well-ordered droplet size distribution. The aerosol formation zone is 1.5 - 2 meters. The disinfected room is saturated with a smooth aerosol of a given density at the required processing time, which ensures effective disinfection even in hard-to-reach places.

Examples of disinfection treatment in various modes are given in TABLE I.

### INDUSTRIAL APPLICABILITY

Thus, the mobile hygienic unit for aerosol disinfection of enclosed spaces allows for highly precise regulation of droplet size distribution of the aerosol in all modes of the aerosol method using any disinfectants, including chlorine dioxide, without changing the spraying nozzle, as well as expanding the range of automated aerosol disinfection equipment.

**TABLE I**

| Object of disinfection | Type of pathogenic micro organisms | Disinfectant | Aerosol mode/ droplet size distribution | Time of action |
|---|---|---|---|---|
| Air environment and surfaces of appliances and furniture in a polyclinic | Bacteria | Chlorine dioxide (concentration 0.12%, consumption 10 ml) | Dry fog | 60 min |
| Air environment and surfaces of appliances and furniture in a maternity hospital | Viruses | Chlorine dioxide (concentration 0.12%, consumption 10 ml) | Wet fog | 30 min |
| Air environment and surfaces of appliances and furniture in an antituberculosis institution | Tuberculosis microbacteria | Chlorine dioxide (concentration 0.2%, consumption 20 ml) | Mist | 60 min |
| Ventilation and air conditioning systems in enclosed spaces | Legionella | Chlorine dioxide (concentration 0.12%, consumption 20 ml) | Dry fog | 60 min |
| Air ducts in enclosed spaces | Legionella | Polyhexamethylene-guanidine (concentration 1.2%, consumption 100 ml) | Spray | 30 min |
| Enclosed spaces in listed buildings (old buildings) | Mold fungi | 1 stage - hydrogen peroxide (concentration 6%, consumption 100 ml); 2 stage - polyhexamethylene-guanidine (concentration 3 .2%, consumption 20 ml) | Spray Mist | 15 min 60 min |
| Air environment and surfaces of appliances and furniture in a multi-specialty hospital | Hospital strains | Sodium dichloroisocyanurate (concentration 0.1%, consumption 20 ml) | Wet fog | 90 min |
| The air environment in infectious diseases hospital | Bacteria in spore form | Hydrogen peroxide (concentration 6%, consumption 20 ml) | Mist | 90 min |

## Claims

1. The mobile hygiene unit comprising a housing containing the spraying unit of a disinfectant with the spraying nozzle and the chamber for mixing the disinfectant with compressed air, the container with the disinfectant, a compressor and the control unit, **characterized in that** it additionally comprises a mechanism for regulating droplet size distribution of the aerosol, including a dozing nozzle with an inner cylinder and a rod with a tip, while the chamber for mixing the disinfectant with compressed air interacts with the dosing nozzle, the inner cylinder of the dosing nozzle being designed so as to move in the chamber for mixing the disinfectant with compressed air. In addition, the spraying nozzle is equipped with the nozzle of axisymmetric cross-section.

2. Mobile hygiene unit according to claim 1, identifiable by antistatic swivel wheels.

3. Mobile hygiene unit according to claim 1, identifiable by a light alarm unit.

4. Mobile hygiene unit according to claim 1, identifiable by a sound alarm unit.

5. Mobile hygiene unit according to claim 1, identifiable by a remote control.

6. Mobile hygiene unite according to claim 1, identifiable by the spraying unit of the disinfectant and the dosing nozzle made of an anticorrosive chemically resistant material.
